(19) Europäisches Patentamt
European Patent Office
Office européen des brevets

(11) **EP 3 186 384 B1**

(12) **FASCICULE DE BREVET EUROPEEN**

(45) Date de publication et mention de la délivrance du brevet:
**26.09.2018 Bulletin 2018/39**

(51) Int Cl.:
***C12P 23/00*** *(2006.01)*   ***C12N 1/12*** *(2006.01)*

(21) Numéro de dépôt: **15771179.7**

(86) Numéro de dépôt international:
**PCT/FR2015/052294**

(22) Date de dépôt: **28.08.2015**

(87) Numéro de publication internationale:
**WO 2016/030644 (03.03.2016 Gazette 2016/09)**

(54) **PROCÉDÉ D'INDUCTION DE LA SYNTHÈSE DES PHYCOBILIPROTEINES**

VERFAHREN ZUR INDUZIERUNG DER SYNTHESE VON PHYCOBILIPROTEINEN

METHOD FOR INDUCING THE SYNTHESIS OF PHYCOBILIPROTEINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2014 FR 1401919**

(43) Date de publication de la demande:
**05.07.2017 Bulletin 2017/27**

(73) Titulaire: **Algobiotech**
**91030 Evry Cedex (FR)**

(72) Inventeurs:
• **BEN OUADA, Hatem**
**91030 EVRY Cedex (TN)**
• **AMMAR, Jihene**
**91030 EVRY Cedex (TN)**

(74) Mandataire: **Marro, Nicolas et al**
**Cabinet Beau de Lomenie**
**158 rue de l'Université**
**75340 Paris Cedex 07 (FR)**

(56) Documents cités:
• **PATEL A ET AL: "Purification and characterization of C-Phycocyanin from cyanobacterial species of marine and freshwater habitat", PROTEIN EXPRESSION AND PURIFICATION, ACADEMIC PRESS, SAN DIEGO, CA, vol. 40, no. 2, 1 avril 2005 (2005-04-01), pages 248-255, XP004781376, ISSN: 1046-5928, DOI: 10.1016/J.PEP.2004.10.028**

• **JELICA SIMEUNOVIC ET AL: "Impact of nitrogen and drought on phycobiliprotein content in terrestrial cyanobacterial strains", JOURNAL OF APPLIED PHYCOLOGY, vol. 25, no. 2, 1 avril 2013 (2013-04-01), pages 597-607, XP055184468, ISSN: 0921-8971, DOI: 10.1007/s10811-012-9894-1**

• **MORENO JOSE ET AL: "Nitrogen-fixing cyanobacteria as source of phycobiliprotein pigments: Composition and growth performance of ten filamentous heterocystous strains", JOURNAL OF APPLIED PHYCOLOGY, vol. 7, no. 1, 1995, pages 17-23, XP008176060, ISSN: 0921-8971**

• **GAURAV SHARMA ET AL: "Effect of Carbon Content, Salinity and pH on Spirulina platensis for Phycocyanin, Allophycocyanin and Phycoerythrin Accumulation", JOURNAL OF MICROBIAL & BIOCHEMICAL TECHNOLOGY, vol. 06, no. 04, 1 janvier 2014 (2014-01-01), pages 202-206, XP055184050, DOI: 10.4172/1948-5948.1000144**

• **R. PRASANNA ET AL: "Rediscovering cyanobacteria as valuable sources of bioactive compounds (Review)", APPLIED BIOCHEMISTRY AND MICROBIOLOGY, vol. 46, no. 2, 1 mars 2010 (2010-03-01), pages 119-134, XP055184829, ISSN: 0003-6838, DOI: 10.1134/S0003683810020018**

• **MAURYA S S ET AL: "Factors regulating phycobiliprotein production in cyanobacteria", INTERNATIONAL JOURNAL OF CURRENT MICROBIOLOGY AND APPLIED SCIENCES, vol. 3, no. 5, avril 2014 (2014-04), pages 764-771, XP055184020, ISSN: 2319-7706**

- **ANUJA A. KENEKAR ET AL: "Effect of Varying Physicochemical Parameters on the Productivity and Phycobiliprotein Content of Indigenous Isolate Geitlerinema sulphureum", BIOTECHNOLOGY(FAISALABAD), vol. 12, no. 3, 1 mars 2013 (2013-03-01), pages 146-154, XP055184036, ISSN: 1682-296X, DOI: 10.3923/biotech.2013.146.154**

**Description**

La présente invention concerne un procédé d'induction de la synthèse de phycobiliprotéines à partir d'une biomasse d'algues.

Plus particulièrement, la présente invention concerne un procédé de préparation d'une biomasse de microalgues photosynthétiques présentant une teneur en phycobiliprotéines au moins égale à 20% du poids sec de ladite biomasse, comprenant les étapes de blocage de la croissance de ladite biomasse et d'induction de la synthèse des phycobiliprotéines.

Les phycobiliprotéines sont des protéines pigmentaires photosynthétiques présentes chez les microalgues des groupes des rhodophycées, des cryptophycées et des cyanobactéries (algues bleues). Ce sont des pigments protéiques ayant une capacité colorante allant du rouge au bleu.

Parmi les phycobiliprotéines, on en distingue la phycocyanine, la phycoérythrine, l'allophycocyanine et la phycoérythrocyanine.

La phycocyanine est un pigment protéique naturel bleu trouvé exclusivement chez les algues bleues: les cyanobactéries. La phycocyanine est un composant de la chaine de transfert d'électrons située dans les phycobilisomes, dont le rôle est la collecte de l'énergie lumineuse dans la zone rouge du spectre visible et son transfert au centre réactionnel du photosystème II du complexe photosynthétique. Dans certaines conditions de stress qui inhibent l'activité photosynthétique, la phycocyanine participe aux mécanismes de protection qui consistent à dissiper l'excès d'énergie par fluorescence. Ces conditions aboutissent à une augmentation de la synthèse de ce pigment et son accumulation sous forme de réserve azotée pour la cellule.

Les facteurs essentiels qui régissent la synthèse de la phycocyanine chez plusieurs espèces de cyanobactéries sont la teneur en azote et en fer, la lumière et la température. Il est démontré que les cyanobactéries peuvent réguler leur teneur en phycocyanine en fonction de ces paramètres dans une gamme de variation propre à chaque espèce (Hemlata, 2009).

La phycocyanine est dotée de propriétés anti-oxydantes et fluorescentes qui lui permettent d'avoir des applications pharmaceutiques et médicales diverses (Erikson, 2008 et Liu *et al,* 2000). Son caractère naturel, son extraction en solution aqueuse et en absence de tout solvant organique en plus de ses propriétés thérapeutiques et de sa couleur bleue unique sont autant d'avantages qui lui permettent de s'intégrer aisément dans les exigences des normes européennes et américaines, liées aux marchés alimentaires et thérapeutiques.

En plus, la résolution des problèmes liés à la stabilité de la phycocyanine en solution aqueuse, à sa forte photosensibilité et à la difficulté de sa conservation, a permis au cours de ces dernières années la prospérité du marché de la phycocyanine de par le monde.

La phycocyanine commercialisée sur le marché mondial est extraite, exclusivement, à partir d'une cyanobactérie : *Arthrospira platensis* (la spiruline). La spiruline est une cyanobactérie produite à grande échelle et a l'avantage de synthétiser jusqu'à 25% de son poids sec en phycocyanine, dans certaines conditions, d'après la littérature.

La production de la phycocyanine est en général affectée par différents facteurs environnementaux (Niels T. Eriksen.2008, Remziye Aysun Kepekçi & Saadet Demirörs Saygideger, 2012). L'azote et la lumière en particulier, sont les facteurs les plus importants. Le premier est nécessaire pour la synthèse des acides aminés constituant les protéines et d'autres composés cellulaires dont les phycocyanines (Samy Boussiba and Amos E. Richmond.1980 and Remziye Aysun Kepekçi & Saadet Demirörs Saygideger, 2012) alors que la luminosité est la source d'énergie pour toute activité photo synthétique

Il est connu de l'homme du métier que la productivité en biomasse est liée à la densité de la culture par une relation de forme parabolique passant par un optimum. La densité optimale favorise le métabolisme primaire et donc l'orientation de l'énergie de la photosynthèse vers la croissance et la reproduction. Lorsque la densité est élevée au-delà d'un certain seuil la croissance s'arrête et l'énergie collectée par la photosynthèse est orientée vers le métabolisme secondaire et donc vers la synthèse de métabolites de défense (ou de stress). Dans certaines conditions de stress (excès d'azote, faible luminosité) les phycocyanines constituent les métabolites de défense les plus synthétisés (Chen *et al.* 1996; Tomaselli *et al.* 1997).

Par ailleurs, dans l'art antérieur, les conditions de culture permettant d'optimiser la synthèse de phycocyanines et celles qui permettent d'optimiser la croissance de la biomasse sont assez opposées. En effet, les conditions optimales de croissance des microalgues varient généralement de 100 à 200 $\mu$mol $m^{-2}$ $s^{-1}$ (3300 à 6600 Lux). Une culture de microalgues réalisée à une intensité lumineuse optimale permettra une rapide croissance de la biomasse mais une synthèse de phycocyanine peu importante. A l'inverse, une culture de microalgues réalisée à de plus faibles intensités lumineuses, (10 à 40 $\mu$mol $m^{-2}$ $s^{-1}$) croit lentement et engendre donc une faible productivité de la biomasse. Cependant, dans ces mêmes conditions de faible intensité lumineuse, les teneurs en phycocyanines peuvent doubler ou tripler de valeur.

Par ailleurs, les plus fortes synthèses de phycocyanine sont obtenues dans des conditions d'excès d'azote et donc des teneurs en azote qui dépassent celles administrées dans les cultures industrielles.

Il existe différents systèmes de production des microalgues, des systèmes dits ouverts, naturels dans des lagunes ou des étangs et des systèmes dits fermés, constitués de photobioréacteurs.

Les systèmes ouverts, le plus souvent des étangs de faible profondeur (30 cm maximum) sont les plus utilisés car ils sont simples à mettre en oeuvre et peu onéreux. Néanmoins, ils ont le désavantage d'être soumis aux variations des conditions climatiques, à l'évaporation de l'eau, et le brassage de la culture est souvent insuffisant pour permettre une bonne exposition à la lumière. Ils présentent ainsi deux principaux inconvénients : l'absence de contrôle de la culture et la faible productivité.

Les systèmes fermés en photobioréacteurs sont des systèmes contrôlés, s'agissant de l'eau ou des nutriments. Néanmoins, ce sont des systèmes très couteux et dont la mise en oeuvre n'est pas facile, notamment en ce qui concerne l'accès à la lumière.

Dans toutes les analyses réalisées par les inventeurs sur de la poudre de spiruline ou sur de la biomasse fraiche provenant de différentes origines et issues de différentes cultures en croissance, que ce soit en étang ou en photobioréacteur, le taux de phycocyanine maximal obtenu est de 12% du poids sec de la biomasse. Les teneurs sont variables d'un échantillon à l'autre et sont comprises entre 3% à 12% du poids sec de la biomasse. Les teneurs en phycobiliprotéines obtenues sont donc toujours en deçà des valeurs maximales (20 à 25% du poids sec) de phycibiliprotéines présentes initialement dans la biomasse; ces valeurs maximales ne sont atteignables que sur des temps de croissance longs.

Sharma et al. 2014 (Journal of Microbial & Biochemical Technology, 6(4):202-206) décrit l'effet de la salinité, du pH et de la source de carbone sur l'accumulation de phycobiliprotéines par Spirulina platensis. Tous les travaux antérieurs visant à augmenter la synthèse des phycocyanines ont été réalisés durant la phase de croissance. Par ailleurs, dans toute la littérature scientifique touchant la synthèse de la phycocyanine les différents facteurs de stress qui favorisent cette synthèse sont étudiés indépendamment les uns des autres.

Dans un processus de production, les facteurs interagissent ensemble d'une manière synergique ou antagoniste sur la synthèse de la phycocyanine, en fonction de l'intensité de chaque facteur. Trouver la zone focale des facteurs de stress, dans un processus multifactoriel, est nécessaire pour maximiser la synthèse de ce métabolite.

En conséquence, le problème technique que cherche à résoudre l'invention est d'optimiser la synthèse des phycobiliprotéines à partir d'une biomasse de microalgues photosynthétiques sans altérer la croissance de ladite biomasse, en définissant des conditions de croissance de la microalgue et de synthèse des phycobiliprotéines optimales.

Le problème technique est résolu par le procédé selon la présente invention qui comprend une étape de blocage de la croissance de la biomasse et une étape d'induction de la synthèse des phycobiliprotéines, ledit procédé étant adaptable à tout système de culture classique de microalgues, compte tenu qu'il est réalisé après récolte d'une partie d'une culture de microalgue.

La présente invention a donc pour objet principal un procédé de préparation d'une biomasse de microalgues photosynthétiques, en particulier de cyanobactéries, plus particulièrement d'*Arthospira Platensis,* comprenant l'induction de la synthèse des phycobiliprotéines dans une biomasse dont la croissance est bloquée. Le procédé de la présente invention permet d'obtenir une biomasse présentant une teneur en phycobiliprotéines au moins égale à 20% du poids sec de ladite biomasse.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

Lors de l'induction, ladite biomasse dont la croissance est bloquée est en contact avec un milieu d'induction, en particulier une solution d'induction.

Le procédé de la présente invention permet en particulier d'obtenir une biomasse présentant une teneur en phycobiliprotéines au moins égale à 21%, 22%, 23%, 24% ou 25% du poids sec de ladite biomasse.

Le procédé de la présente invention permet en particulier d'obtenir une biomasse présentant une teneur en phycobiliprotéines comprise de 20% à 25% du poids sec de ladite biomasse.

Le procédé selon l'invention étant réalisé en dehors du bassin de culture, il permet de ne pas altérer la productivité de la biomasse.

On entend par « biomasse », la masse totale des microalgues vivantes à un moment donné dans une culture. Elle est exprimée en g/L.

On entend par « phycobiliprotéines », les protéines pigmentaires hydrosolubles de la photosynthèse telles que l'allophycocyanine (APC), la famille des phycocyanines (C-PC et R-PC), la famille des phycoérythrines (R-PE et C-PE) et la phycoérythrocyanine (PEC), isolées de certaines microalgues ou de cyanobactéries.

On entend par « induction de la synthèse », la stimulation par tout moyen de la production de phycobiliprotéines par une biomasse de microalgues.

On entend par « milieu d'induction » tout milieu de culture de microalgues contenant un ou plusieurs sels en excès ou en déficit par rapport à la concentration en sels nécessaire à la croissance optimale. Dans le cadre de l'invention ce milieu contient un excès d'azote obtenu par l'ajout de $NaNO_3$ au milieu de culture Zarrouk, à une concentration supérieure à 2,5 g/L et de préférence comprise de 3 à 4 g/L.

On entend par « blocage de croissance », le fait de stopper la multiplication de la microalgue. Le blocage de la croissance est réversible. En effet, suite au blocage de la croissance, une partie de la biomasse est collectée. La concentration est alors ainsi diminuée et la biomasse peut de nouveau croitre. La biomasse n'est donc pas altérée. La détermination du blocage de croissance (le fait que la multiplication des microalgues soit bloquée) se fait par exemple en suivant la courbe de vitesse de croissance des microalgues en fonction de la densité de culture. Un blocage est observé lorsque la vitesse de croissance des microalgues est nulle.

Dans un mode de réalisation de l'invention, le procédé se caractérise en ce que le blocage de la croissance de ladite biomasse de microalgues, en particulier de cyanobactéries, plus particulièrement d'*Arthospira Platensis,* est réalisé dans un bassin d'induction, ladite concentration de ladite biomasse dans ledit bassin étant de 3 à 13 fois plus élevée que la concentration permettant la croissance optimale des microalgues en culture.

Dans un mode de réalisation de l'invention, le procédé se caractérise en ce que le blocage de la croissance de ladite biomasse de microalgues, en particulier de cyanobactéries, plus particulièrement d'*Arthospira Platensis,* est réalisé dans un bassin d'induction, ladite concentration de ladite biomasse dans ledit bassin étant 3, 4, 5, 6, 7, 8, 9, 10, 11, 12 ou 13 fois plus élevée que la concentration permettant la croissance optimale des microalgues en culture.

Le terme « concentration » peut être remplacé par le terme « densité ». On parle indifféremment de « densité de la biomasse » ou de « concentration de la biomasse ». La densité comme la concentration sont exprimées en g/l.

Il est admis que la densité/concentration permettant la croissance optimale des microalgues en culture est d'environ 0,4 g/l.

Ce blocage est réalisé dans le cadre de l'invention par l'introduction de la biomasse à une concentration allant de 1 à 5 g/l dans un bassin d'induction. La concentration de ladite biomasse au moment du blocage peut être égale à 1 g/l, 1,5 g/l, 2 g/l, 2,5 g/l, 3 g/l, 3,5 g/l, 4 g/l, 4,5 g/l ou encore 5 g/l.

La densité/concentration de la biomasse peut être mesurée par différentes méthodes.

- Le disque de Secchi : C'est un dispositif simple permettant d'estimer rapidement la densité des cellules cultivées en milieu aquatique. Il est constitué d'une règle graduée de 30cm de long, munie d'un disque blanc de 5cm de diamètre à l'extrémité inférieure, au point zéro. On note la profondeur, en centimètres, à partir de laquelle on ne peut plus distinguer le disque une fois plongé dans le milieu.

- Les comptages sous microscope. Cette méthode est coûteuse en temps, mais elle permet d'estimer le nombre de filaments et le nombre de spires par filament. A l'aide d'une pipette calibrée, on dépose une goutte d'échantillon sur une lame creuse pour l'observation sous microscope; on évalue le nombre de filaments dans une goutte, sachant que 17 gouttes de notre pipette représentent un volume de 1 ml. Si la culture est très concentrée, on dilue l'échantillon. Le nombre moyen de spires est calculé sur 30 filaments pris au hasard.

- La densité optique à 665 nm. Cette méthode permet d'estimer rapidement la biomasse en utilisant l'absorption à 665 nm, qui est une des longueurs d'onde d'absorption de la chlorophylle. Cette absorption in vivo est généralement bien corrélée à la concentration en chlorophylle. On utilise un spectrophotomètre équipé d'une cuve à faces parallèles de 25 cl. Le zéro est fait sur du milieu de culture non ensemencé.

Le bassin d'induction peut être un bassin de culture intermédiaire ou un bassin de récupération ou tout autre système clos de stockage de microalgues vivantes. Dans un mode de réalisation préféré de l'invention, la concentration de ladite biomasse dans le bassin d'induction est comprise entre une valeur sensiblement égale ou supérieure à 1g/L et une valeur sensiblement égale ou inférieure à 5 g/L. Cette concentration est obtenue en additionnant du milieu d'induction dans le bassin d'induction.

La concentration de 1g/L à 5g/L correspondant à une densité de 3 à 13 fois supérieure à la densité de la biomasse en croissance au moment de la collecte, c'est-à-dire au moment de la croissance optimale où la concentration est d'environ 0,4g/l. A cette concentration, la croissance est bloquée pour favoriser le métabolisme de défense de la microalgue.

La croissance d'une biomasse, par exemple en croissance optimale, notamment à une concentration d'environ 0,4g/l peut être bloquée en portant ladite biomasse à une concentration comprise de 1g/L à 5g/L par exemple par filtration, décantation ou centrifugation.

La biomasse peut également subir un lavage avant son dépôt dans le bassin d'induction. Le lavage permet d'éliminer les sels en excès provenant du milieu de culture. Ceci permet ainsi un meilleur contrôle de la concentration en azote du milieu, en particulier de la solution, d'induction.

Dans ce cas la biomasse est lavée 3 fois en utilisant une eau physiologique ou la solution du milieu d'induction fraichement préparée.

Dans ces conditions, la biomasse initiale n'est pas affectée par le procédé et l'induction de la synthèse des phycocyanines n'est pas dépendante des conditions de culture de la biomasse.

Cette procédure présente l'avantage de pouvoir être facilement intégrée dans la chaine de production d'une ferme traditionnelle de culture de spiruline. Elle peut être appliquée dans un bassin de culture intermédiaire ou dans un bassin de récupération pour une durée de 3 heures, 4 heures ou jusqu'à 5 heures.

Dans un mode de réalisation particulier de l'invention, l'étape d'induction de la synthèse des phycobiliprotéines comprend :

1) l'exposition de la biomasse de microalgues photosynthétiques, en particulier de cyanobactéries, plus particulièrement d'*Arthospira Platensis,* à un flux lumineux, ledit flux ayant une intensité lumineuse comprise d'une valeur sensiblement égale ou supérieure à 10 $\mu$mol $m^{-2} s^{-1}$ à une valeur sensiblement égale ou inférieure à 13 $\mu$mol $m^{-2} s^{-1}$ ;
2) l'ajout d'une source d'azote de manière à obtenir une concentration en $NaNO_3$ dans le milieu d'induction supérieure à 2,5 g/L.

L'intensité lumineuse en $\mu$moles de photons par mètre carré et par seconde, est par exemple déterminée à l'aide d'un photomètre mesurant les radiations actives pour la photosynthèse.

A noter que 10 $\mu$mol $m^{-2} s^{-1}$ correspondent à 330 Lux et que 13 $\mu$mol $m^{-2} s^{-1}$ correspondent à 429 Lux, en particulier avec pour éclairage un tube fluorescent, par exemple de type « Plant Growth fluorescent ». Dans ce cas, 1 $\mu$mole de photons par mètre carré et par seconde équivaut à 33 lux.

La concentration en $NaNO_3$ dans le milieu d'induction est notamment comprise de 3 à 4g/L ou de 3 à 5g/l.

La combinaison d'une intensité lumineuse comprise dans un intervalle allant de 10 à 13 $\mu$mol $m^{-2} s^{-1}$ et d'une solution de culture contenant 3 à 5g/L de $NaNO_3$ (comme source d'azote) permet l'obtention d'une biomasse contenant de 20 à 25% de phycocyanine, ce qui est particulièrement élevée par rapport à l'intervalle de 3 à 12% obtenu dans les expériences mises en oeuvre sans application du procédé de l'invention.

La lumière peut être assurée par des lampes à incandescence simple de puissance 40W ou par des tubes fluorescents.

Le milieu d'induction préférée est constitué par le milieu de culture Zarrouk, mais tout autre milieu de culture adapté à la spiruline peut être utilisé. Dans tous les cas, la concentration en azote du milieu, en particulier de la solution, d'induction est modifiée par l'ajout de nitrate de sodium ($NaNO_3$) en excès. La concentration finale doit être comprise dans un intervalle allant de 3 à 5g/l de $NaNO_3$, ce qui représente une concentration 1,2 à 2 fois supérieure à celle présente dans le milieu Zarouk initial (2,5g/l de $NaNO_3$).

Dans les conditions de culture définies ci-dessus, l'étape d'induction est réalisée pendant une durée de 3 heures, 4 heures ou jusqu'à 5 heures et est suffisante pour induire la synthèse des phycobiliprotéines à une teneur stable comprise entre 20 et 25% de la biomasse sèche.

Le procédé selon l'invention, de préparation d'une biomasse de microalgues photosynthétiques, en particulier de cyanobactéries, plus particulièrement d'*Arthospira Platensis,* présentant une teneur en phycobiliprotéines au moins égale à 20% du poids sec de ladite biomasse, comprend une étape de blocage de la croissance de la biomasse et une étape d'induction de la synthèse des phycobiliprotéines.

Selon un mode de réalisation avantageux, la biomasse présente une teneur en phycobiliprotéines au moins égale à 21%, 22%, 23%, 24% ou 25% du poids sec de ladite biomasse.

Selon un mode de réalisation avantageux, la biomasse présente une teneur en phycobiliprotéines comprise de 20% à 25% du poids sec de ladite biomasse.

Dans un mode de réalisation particulier, le procédé de la présentation invention, peut comprendre une étape préalable de collecte d'une biomasse de microalgues, en particulier de cyanobactéries, plus particulièrement d'*Arthospira Platensis.* La biomasse est collectée dans des bassins de cultures usuels, lorsque la culture est en phase exponentielle de croissance, c'est à dire lorsque la productivité en biomasse est maximale. Le volume récupéré est filtré sur toile (ou par décantation ou par centrifugation) pour obtenir la biomasse fraiche. La biomasse obtenue est transférée dans le bassin d'induction.

Le procédé selon l'invention peut également comprendre une étape finale de collecte de la biomasse enrichie.

Le procédé selon l'invention peut donc comprendre les étapes de

- a) collecte d'une biomasse de microalgues, en particulier de cyanobactéries, plus particulièrement d'*Arthospira Platensis*
- b) blocage de la croissance de ladite biomasse
- c) induction de la synthèse des phycobiliprotéines
- d) collecte de ladite biomasse enrichie en phycobiliprotéines

Le procédé selon l'invention utilise les microalogues photosynthétiques choisies parmi les cyanobactéries, les rhodophytes ou les cryptophytes.

De manière préférée, la microalgue photosynthétique utilisée pour mettre en oeuvre le procédé selon l'invention est *Arthospira Platensis,* aussi appelée spiruline.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

Les phycobiliprotéines synthétisées dans le cadre du présent procédé peuvent être de la phycocyanine, de l'allophycocyanine, de la phycoérythrine ou de la phycoérythrocyanine ou un mélange d'au moins deux de ces phycobiliprotéines.

La biomasse de microalgues photosynthétiques, en particulier de cyanobactéries, plus particulièrement d'*Arthospira Platensis,* présentant une teneur en phycobiliprotéines au moins égale à 20% du poids sec de ladite biomasse, est susceptible d'être obtenue par le procédé précédemment décrit. La biomasse présente une teneur en phycobiliprotéines au moins égale à 21%, 22%, 23%, 24% ou 25% du poids sec de ladite biomasse.

Plus particulièrement, la biomasse de microalgues photosynthétiques est une biomasse extraite à partir d'algues *Arthospira Platensis.* Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

La biomasse de microalgues photosynthétiques, en particulier de cyanobactéries, plus particulièrement d'*Arthospira Platensis,* susceptible d'être obtenue par le procédé de la présente invention, peut être utilisée comme complément alimentaire dans le cadre d'une préparation de produit nutraceutique ou comme constituant de préparation cosmétiques.

On entend par produit nutraceutique, tout produit fabriqué à partir de substances alimentaires, mais rendu disponible sous forme de comprimé, de poudre, de boisson, ou d'autre formes médicinales habituellement non associées à des aliments, et qui s'est avéré avoir un effet physiologique bénéfique ou protecteur contre des maladies.

Une composition cosmétique, nutraceutique ou à composition à visée de complément alimentaire, est préparée à partir d'une biomasse ou d'un mélange de biomasses de microalgues photosynthétiques, en particulier de cyanobactéries, plus particulièrement *d'Arthospira Platensis,* présentant une teneur en phycobiliprotéines au moins égale à 20% du poids sec de ladite biomasse.

La biomasse présente une teneur en phycobiliprotéines au moins égale à 21%, 22%, 23%, 24% ou 25% du poids sec de ladite biomasse.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

Ladite composition cosmétique, nutraceutique ou composition à visée de complément alimentaire est de préférence préparée à partir d'une biomasse d'algues *Arthospira Platensis.*

La composition cosmétique, possède des propriétés antirides intensives permettant de lutter contre les rides profondes, le relâchement cutané et le teint terme.

La composition cosmétique est riche en composés anti-oxydants. Elle agit contre les radicaux livres et stimulent l'oxygénation cellulaire. Avec une application continue, le contour du visage est redessiné, les rides profondes sont réduites et le teint est plus uniforme. La composition cosmétique permet aussi de réduire les poches et cernes sous les yeux et protège de la déshydratation pour un meilleur confort.

La composition cosmétique, a également des effets anti-tâches et stimule la détoxification cellulaire, protège des effets du soleil et de la pollution, accroit la capacité de lutte contre les taches caractérisées par un excès de mélanine.

Selon l'intensité des taches, deux à quatre applications quotidiennes sont nécessaires. Les taches claires et diffuses disparaissent en quelques semaines alors que les taches foncées et à contour défini peuvent nécessiter 6 à 12 mois pour disparaitre complètement. La composition cosmétique est également conseillée pour les taches résiduelles des fortes poussées d'acné. Par ailleurs, elle a un effet hydratant pour une peau souple et douce.

A la fin du traitement de la biomasse selon le procédé de l'invention, ladite biomasse enrichie en phycobiliprotéines peut être filtrée et lavée. Elle peut être incorporée dans le circuit d'essorage et de séchage de la chaine de production. Elle peut être commercialisée dans des formulations parapharmaceutiques comme par exemple des suppléments alimentaires destinés à renforcer le système immunitaire chez le nourrisson ou des produits cosmétiques à fort pouvoir antioxydant.

La biomasse peut également servir à l'extraction et à la clarification d'une ou plusieurs phycobiliprotéines. Par exemple, la phycocyanine produite peut être concentrée et stabilisée et être ainsi introduite dans des formulations alimentaires cosmétiques ou médicales.

Une composition dermatologique peut contenir à titre de substance active une biomasse ou un mélange de biomasses de microalgues photosynthétiques, en particulier de cyanobactéries, plus particulièrement d'*Arthospira Platensis,* présentant une teneur en phycobiliprotéines au moins égale à 20% du poids sec de ladite biomasse, telle que définie précédemment, et un véhicule pharmaceutiquement acceptable.

La biomasse présente une teneur en phycobiliprotéines au moins égale à 21%, 22%, 23%, 24% ou 25% du poids sec de ladite biomasse.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

L'utilisation d'une biomasse ou d'un mélange de biomasses de microalgues photosynthétiques, en particulier de cyanobactéries, plus particulièrement *d'Arthospira Platensis,* présentant une teneur en phycobiliprotéines au moins égale à 20% du poids sec de ladite biomasse, telle que définie précédemment, en tant qu'antioxydant est décrit.

La biomasse présente une teneur en phycobiliprotéines au moins égale à 21%, 22%, 23%, 24% ou 25% du poids sec de ladite biomasse.

Lesdites microalgues photosynthétiques sont en particulier choisies parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

## DESCRIPTION DES TABLEAUX ET FIGURES

Le tableau 1 représente la composition d'une solution d'induction utilisable dans le cadre de l'invention, et comprenant 4g/L de $NaNO_3$.

La Tableau 2 représente la matrice expérimentale : la combinaison des trois niveaux correspondant aux deux facteurs, luminosité et concentration en azote, ainsi que le rendement en phycobiliprotéines obtenu exprimé en pourcentage de poids sec.

La figure 1 représente les courbes d'iso-rendement de la synthèse de la phycocyanine en fonction de l'intensité lumineuse ($\mu$mol $m^{-2} s^{-1}$) et de la concentration en azote (g/l) pour une durée d'induction de 3 heures.

La figure 2 représente les courbes d'iso-rendement de la synthèse de la phycocyanine en fonction de l'intensité lumineuse ($\mu$mol $m^{-2} s^{-1}$) et de la concentration en azote (g/l) pour une durée d'induction de 24 heures.

La figure 3 représente les courbes d'iso-rendement de la synthèse de la phycocyanine en fonction de l'intensité lumineuse ($\mu$mol $m^{-2} s^{-1}$) et de la concentration en azote (g/l) pour une durée d'induction de 3 jours.

## EXEMPLES:

### Exemple 1 : synthèse de la phycocyanine

La variation du rendement de synthèse de la phycocyanine est étudiée pour différentes concentrations en azote et à différentes intensités lumineuses.

L'étude est réalisée selon la méthodologie des plans d'expérience, moyennant un plan factoriel à trois niveaux avec interaction (Goupy, 2001).

La biomasse fraiche est obtenue à partir d'une culture en phase exponentielle de la cyanobactérie photosynthétique *Arthospira Platensis,* concentrée 5 fois. La solution d'induction utilisée est le milieu Zarouk modifié : la concentration en $NaNO_3$ comme source d'azote de 1 à 4 g/l. La biomasse concentrée dans le milieu d'induction est soumise à différentes intensités lumineuses dans un intervalle compris entre 10 à 50 $\mu$mol $m^{-2} s^{-1}$.

Onze expériences sont réalisées dont neuf représentent la combinaison entre les trois niveaux correspondant aux deux facteurs (minimum, maximum et milieu). Les trois autres représentent les points centraux et permettent d'évaluer l'erreur expérimentale. La matrice expérimentale est représentée dans le tableau n°2.

Pour chaque condition, trois prélèvements sont réalisés à des périodes différentes : 3 heures, un jour et 3 jours de traitement.

La phycocyanine est extraite à la fin de chacune des 11 conditions expérimentales. L'extraction est refaite deux fois de manière à extraire la totalité de la phycocyanine. Ensuite le rendement en phycocyanine (pourcentage de phycocyanine par gramme de matière sèche) est calculé.

Le rendement de synthèse de la phycocyanine (Ren Phy) est modélisé en fonction de la concentration de l'azote $NaNO_3$ (N) et de l'intensité lumineuse (Lum).

Les différents traitements montrent que l'induction de 3 heures donne les rendements les plus élevés. Le modèle obtenue dans ces conditions est exprimé par l'équation suivante :

$$\textbf{RenPhy \%}^{(2)} = 65 - 45*\textbf{Lum} + 458*\textbf{N} + 1.02*\textbf{Lum}^2 - 51*\textbf{N}^2 - 3.14*\textbf{Lum}*\textbf{N}$$

**EP 3 186 384 B1**

Le modèle est valide à 95%, avec un coefficient de corrélation ($R^2$) de 95%.

L'erreur standard est de 15%.

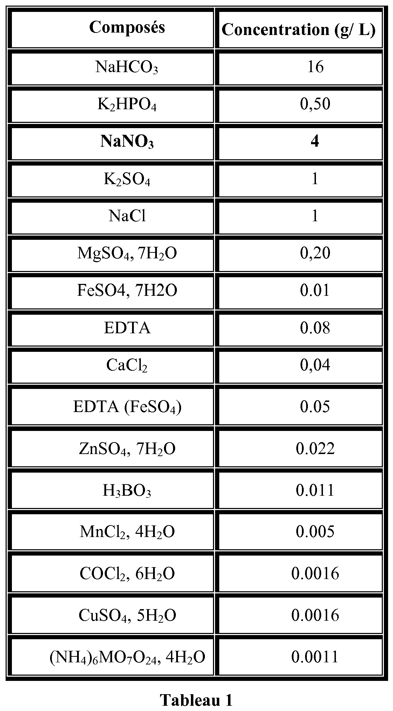

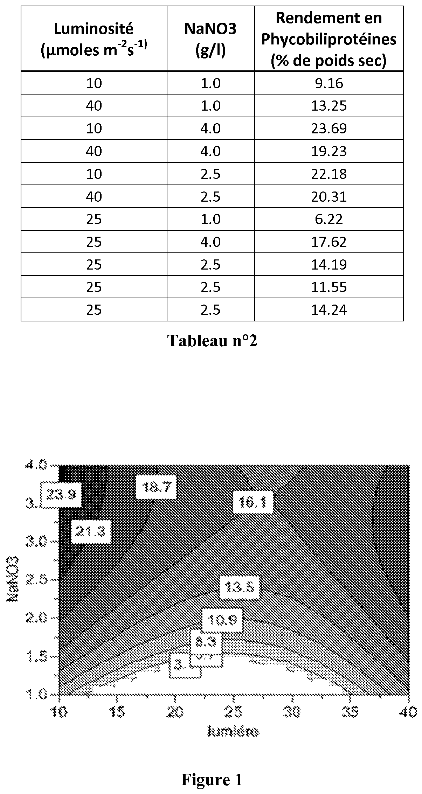

Les courbes d'iso-rendement de la synthèse de la phycocyanine en fonction de l'intensité lumineuse ($\mu$mol $m^{-2}\,s^{-1}$) et de la concentration en NaNO$_3$ (g/L), et obtenues après 3 heures de traitement, selon le procédé de la présente invention, sont illustrées sur la figure 1. Une intensité lumineuse de 10 $\mu$mol $m^{-2}\,s^{-1}$ et une concentration en NaNO$_3$ de 4 g/L permet d'obtenir un rendement en phycocyanine de 22% à 24% par gramme de matière sèche.

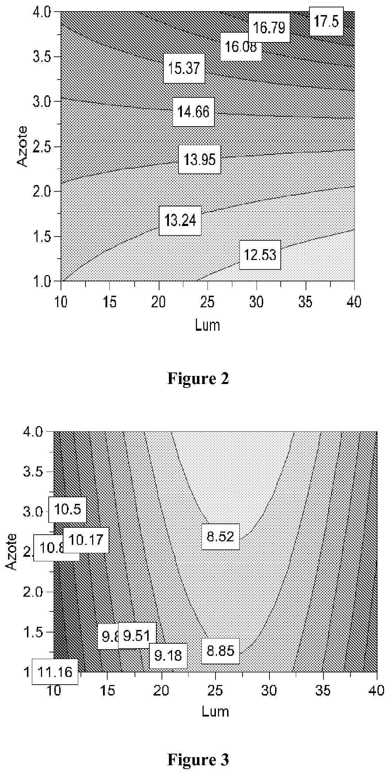

Lorsque l'induction de la synthèse est prolongée à 1 jour les rendements de phycocyanine ne dépassent pas 18% au maximum (figure 2). Un traitement de 3 jours ne permet d'obtenir qu'un rendement maximum inférieur à 12% (figure 3).

**Exemple 2 : Crème anti rides**

Une crème anti rides est obtenue avec la composition suivante :

Aqua: 59%,
Huile d'amande douce: 18%,
Phycobiliprotéines solubilisée dans la glycérine : 7%,
Huile de palme: 5%, Huile de ricin: 3%,
Extrait d'algues: 3% (beta carotène),
Cire d'abeille: 2%,
gomme xanrthane: 1,7%,
Parfum: 1%, éthyl parabène: 0,1%, méthyl parabène: 0,1%, benzyl parabène : 0,1%.

**Exemple 3 : Crème anti taches**

Une crème anti taches est obtenue avec la composition suivante :

Crème anti taches:
Aqua: 59%,
Huile d'amande douce: 19,5%,
Phycobiliprotéines solubilisée dans la glycérine : 7%,
Huile de palme: 5%, Huile de ricin: 3%,
Extrait d'algues: 1,5% (beta carotène),
Cire d'abeille: 2%,
gomme xanrthane: 1,7%,
Parfum: 1%,
éthyl parabène: 0,1%, méthyl parabène: 0,1%, benzyl parabène : 0,1%.

**REFERENCES**

Erikson N.T, 2008, production of phycocyanin, a pigment with applications in biology, biotechnology, foods and medcine. Appl Microbiol Biotechnol, 80, 1-14.

Hemlata T.F, 2009, Screening of cyanobacteria for phycobiliproteins and effect of different environmental Stress on its yield. Bull Environ Contam Toxicol, 83, 509-515. Liu Y, Xu L, Cheng N, Lin L, Zheng Ch, 2000,Inhibitory effect of phycocyanin from Arthrospira platensis on the growth of human leukemia K562 cells. journal of applied phycology, 12, 125-130.

Chen F, Zhang Y and GuoS.1996. Growth and phycocyanin formation of Spirulina platensis in photoheterotrophic culture. Biotechnology Letters, 18, Issue 5, pp 603-608

Remziye Aysun Kepekçi & Saadet Demirörs Saygideger. 2012. Enhancement of phenolic compound production in Spirulina platensis by two-step batch mode cultivation. J Appl Phycol, 24:897-905.

Goupy J., 2001, Introduction aux plans d'expériences, seconde édition Dunod,

**Revendications**

**1.** Procédé de préparation d'une biomasse de microalgues photosynthétiques, en particulier de cyanobactéries, plus particulièrement d'*Arthospira Platensis,* présentant une teneur en phycobiliprotéines au moins égale à 20% du poids sec de ladite biomasse, comprenant l'induction de la synthèse des phycobiliprotéines dans une biomasse dont la croissance est bloquée, **caractérisé en ce que** le blocage de la croissance de ladite biomasse de microalgues est réalisé en concentrant ladite biomasse dans un bassin d'induction, ladite concentration de ladite biomasse dans ledit bassin étant de 3 à 13 fois plus élevée que la densité permettant la croissance optimale des microalgues en culture, soit 0,4g/l.

2. Procédé selon la revendication 1, **caractérisé en ce que** ladite concentration de ladite biomasse est comprise de 1g/L à 5 g/L.

3. Procédé selon la revendication 1, **caractérisé en ce que** l'induction de la synthèse des phycobiliprotéines comprend :

   - l'exposition de la biomasse de microalgues photosynthétiques à un flux lumineux, ledit flux ayant une intensité lumineuse comprise d'une valeur sensiblement égale ou supérieure à 10 micromoles m$^{-2}$ s$^{-1}$ ($\mu$mol *m$^{-2}$ s$^{-1}$*) à une valeur sensiblement égale ou inférieure à 13 $\mu$mol *m$^{-2}$ s$^{-1}$*
   - l'ajout d'une source d'azote de manière à obtenir une concentration en NaNO$_3$ dans ladite biomasse supérieure à 2,5 g/L de NaNO$_3$.

4. Procédé selon la revendication 3, **caractérisé en ce que** l'étape d'induction est réalisée pendant une durée de 3 heures, 4 heures ou 5 heures.

5. Procédé selon la revendication 1 de préparation d'une biomasse de microalgues photosynthétiques présentant une teneur en phycobiliprotéines au moins égale à 20% du poids sec de ladite biomasse , comprenant les étapes suivantes :

   - a) blocage de la croissance de ladite biomasse
   - b) induction de la synthèse des phycobiliprotéines

6. Procédé de préparation d'une biomasse de microalgues photosynthétiques selon la revendication 5, **caractérisé en ce qu'**il comprend une étape préalable de collecte d'une biomasse de microalgues

7. Procédé selon la revendication 5 ou la revendication 6, **caractérisé en ce qu'**il comprend en outre une étape de collecte d'une biomasse enrichie en phycobiliprotéines.

8. Procédé selon la revendication 1, comprenant les étapes suivantes :

   - a) collecte d'une biomasse de microalgues
   - b) blocage de la croissance de ladite biomasse
   - c) induction de la synthèse des phycobiliprotéines
   - d) collecte de ladite biomasse enrichie en phycobiliprotéines.

9. Procédé selon l'une des revendications précédentes, dans lequel les microalgues photosynthétiques sont choisies parmi les cyanobactéries, les rhodophytes ou les cryptophytes.

10. Procédé selon l'une des revendications précédentes, dans lequel la microalgue photosynthétique est choisie parmi les rhodophycées, les cryptophycées et les cyanobactéries, plus particulièrement les cyanobactéries, encore plus particulièrement *Arthospira Platensis* et *Aphanizomenon flos-aquae,* notamment l'algue Klamath.

11. Procédé selon l'une des revendications précédentes, **caractérisé en ce que** les phycobiliprotéines sont choisies parmi la phycocyanine, l'allophycocyanine, la phycoérythrine ou la phycoérythrocyanine ou un mélange d'au moins deux de ces phycobiliprotéines.

12. Procédé selon la revendication 3, dans lequel l'azote est apporté par un milieu de culture comprenant une concentration finale supérieure à 2,5 g/L et inférieure à 5g/L de NaNO$_3$, en particulier le milieu de Zarouk.

**Patentansprüche**

1. Verfahren zur Herstellung einer Biomasse aus photosynthetisierenden Mikroalgen, insbesondere Cyanobakterien, noch spezieller *Arthospira Platensis,* die einen Gehalt an Phycobiliproteinen mindestens gleich 20 % des Trockengewichts der Biomasse aufweisen, umfassend die Induzierung der Synthese der Phycobiliproteine in einer Biomasse, deren Wachstum blockiert ist, **dadurch gekennzeichnet, dass** das Blockieren des Wachstums der Biomasse aus Mikroalgen erfolgt, indem die Biomasse in einem Induktionsbecken konzentriert wird, wobei die Konzentration der Biomasse in dem Becken 3 bis 13 Mal höher ist als die Dichte, die das optimale Wachstum der Mikroalgen in Kultur, also 0,4 g/L, ermöglicht.

2. Verfahren nach Anspruch 1, **dadurch gekennzeichnet, dass** die Konzentration der Biomasse zwischen 1 g/L und 5 g/L beträgt.

3. Verfahren nach Anspruch 1, **dadurch gekennzeichnet, dass** die Induzierung der Synthese der Phycobiliproteine umfasst:

   - die Exposition der Biomasse aus photosynthetisierenden Mikroalgen gegenüber einem Lichtstrom, wobei der Strom eine Leuchtintensität zwischen einem Wert im Wesentlichen gleich oder höher als 10 Mikromol $m^{-2}$ $s^{-1}$ ($\mu$mol $m^{-2}$ $s^{-1}$) und einem Wert im Wesentlichen gleich oder niedriger als 13 $\mu$mol $m^{-2}$ $s^{-1}$ aufweist
   - die Zugabe einer Stickstoffquelle, um eine Konzentration von $NaNO_3$ in der Biomasse von mehr als 2,5 g/L $NaNO_3$ zu erhalten.

4. Verfahren nach Anspruch 3, **dadurch gekennzeichnet, dass** der Schritt der Induzierung über eine Dauer von 3 Stunden, 4 Stunden oder 5 Stunden ausgeführt wird.

5. Verfahren nach Anspruch 1 zur Herstellung einer Biomasse aus photosynthetisierenden Mikroalgen, die einen Gehalt an Phycobiliproteinen mindestens gleich 20 % des Trockengewichts der Biomasse aufweist, umfassend die folgenden Schritte:

   - a) Blockieren des Wachstums der Biomasse
   - b) Induzierung der Synthese von Phycobiliproteinen

6. Verfahren zur Herstellung einer Biomasse aus photosynthetisierenden Mikroalgen nach Anspruch 5, **dadurch gekennzeichnet, dass** es einen vorausgehenden Schritt des Sammelns einer Biomasse aus Mikroalgen umfasst.

7. Verfahren nach Anspruch 5 oder Anspruch 6, **dadurch gekennzeichnet, dass** es ferner einen Schritt des Sammelns einer Biomasse umfasst, die mit Phycobiliproteinen angereichert ist.

8. Verfahren nach Anspruch 1, umfassend die folgenden Schritte:

   - a) Sammeln einer Biomasse aus Mikroalgen
   - b) Blockieren des Wachstums der Biomasse
   - c) Induzierung der Synthese von Phycobiliproteinen
   - d) Sammeln der mit Phycobiliproteinen angereicherten Biomasse.

9. Verfahren nach einem der vorhergehenden Ansprüche, wobei die photosynthetisierenden Mikroalgen ausgewählt sind aus den Cyanobakterien, den Rhodophyten und den Cryptophyten.

10. Verfahren nach einem der vorhergehenden Ansprüche, wobei die photosynthetisierende Mikroalge ausgewählt ist aus den Rhodophyceen, den Cryptophyceen und den Cyanobakterien, insbesondere den Cyanobakterien, noch spezieller *Arthospira Platensis* und *Aphanizomenon flos-aquae,* insbesondere die Alge Klamath.

11. Verfahren nach einem der vorhergehenden Ansprüche, **dadurch gekennzeichnet, dass** die Phycobiliproteine ausgewählt sind aus Phycocyanin, Allophycocyanin, Phycoerythrin oder Phycoerythrocyanin, oder einer Mischung von zumindest zwei dieser Phycobiliproteine.

12. Verfahren nach Anspruch 3, wobei der Stickstoff durch ein Kulturmedium beigebracht wird, das eine Endkonzentration von mehr als 2,5 g/L und weniger als 5 g/L $NaNO_3$ umfasst, insbesondere Zarrouks Medium.

**Claims**

1. A method for preparing a biomass of photosynthetic microalgae, in particular cyanobacteria, more particularly *Arthrospira platensis,* having a content of phycobiliproteins at least equal to 20% of the dry weight of said biomass, comprising inducing the synthesis of phycobiliproteins in a biomass, the growth of which is blocked, wherein **characterized in that** the growth of said biomass of microalgae is blocked by concentrating said biomass in an induction tank, said concentration of said biomass in said tank being from 3 to 13 times higher than the density enabling the optimum growth of microalgae in culture, i.e. 0.4 g/L.

2. The method as claimed in claim 1, wherein said concentration of said biomass is from 1 g/L to 5 g/L.

3. The method as claimed in claim 1, wherein the inducing the synthesis of phycobiliproteins comprises:

   - exposing the biomass of photosynthetic microalgae to a light flux, said flux having a light intensity of a value from substantially equal to or greater than 10 micromoles m$^{-2}$ s$^{-1}$ ($\mu$mol m$^{-2}$s$^{-1}$) to a value substantially equal to or less than 13 $\mu$mol m$^{-2}$ s$^{-1}$
   - adding a source of nitrogen so as to obtain a concentration of NaNO$_3$ in said biomass of greater than 2.5 g/L of NaNO$_3$.

4. The method as claimed in claim 3, wherein the induction step is carried out for a duration of 3 hours, 4 hours or 5 hours.

5. The method as claimed in claim 1 for preparing a biomass of photosynthetic microalgae having a content of phycobiliproteins at least equal to 20% of the dry weight of said biomass, comprising the following steps:

   a) blocking the growth of said biomass
   b) inducing the synthesis of phycobiliproteins.

6. The method for preparing a biomass of photosynthetic microalgae as claimed in claim 5, comprising a prior step of collecting a biomass of microalgae.

7. The method as claimed in claim 5, comprising a step of collecting a biomass enriched in phycobiliproteins.

8. The method as claimed in claim 1, comprising the following steps:

   a) collecting a biomass of microalgae
   b) blocking the growth of said biomass
   c) inducing the synthesis of phycobiliproteins
   d) collecting said biomass enriched in phycobiliproteins.

9. The method as claimed in one of the preceding claims, in which the photosynthetic microalgae are chosen from one of cyanobacteria, rhodophytes and cryptophytes.

10. The method as claimed in one of the preceding claims, in which the photosynthetic microalga is chosen from rhodophyceae, cryptophyceae and cyanobacteria, more particularly cyanobacteria, even more particularly *Arthrospira platensis* and and *Aphanizomenon flos-aquae,* in particular Klamath alga.

11. The method as claimed in one of the preceding claims, **characterized in that** the phycobiliproteins are chosen from phycocyanin, allophycocyanin, phycoerythrin or phycoerythrocyanin, or a mixture of at least two of these phycobiliproteins.

12. The method as claimed in claim 3, in which the nitrogen is provided by a culture medium comprising a final concentration of greater than 2.5 g/L and less than 5 g/L of NaNO$_3$., in particular Zarouk medium.

| Composés | Concentration (g/ L) |
|:---:|:---:|
| $NaHCO_3$ | 16 |
| $K_2HPO_4$ | 0,50 |
| **$NaNO_3$** | **4** |
| $K_2SO_4$ | 1 |
| NaCl | 1 |
| $MgSO_4, 7H_2O$ | 0,20 |
| $FeSO4, 7H2O$ | 0.01 |
| EDTA | 0.08 |
| $CaCl_2$ | 0,04 |
| EDTA ($FeSO_4$) | 0.05 |
| $ZnSO_4, 7H_2O$ | 0.022 |
| $H_3BO_3$ | 0.011 |
| $MnCl_2, 4H_2O$ | 0.005 |
| $COCl_2, 6H_2O$ | 0.0016 |
| $CuSO_4, 5H_2O$ | 0.0016 |
| $(NH_4)_6MO_7O_{24}, 4H_2O$ | 0.0011 |

**Tableau 1**

| Luminosité (μmoles m$^{-2}$s$^{-1)}$ | NaNO3 (g/l) | Rendement en Phycobiliprotéines (% de poids sec) |
|---|---|---|
| 10 | 1.0 | 9.16 |
| 40 | 1.0 | 13.25 |
| 10 | 4.0 | 23.69 |
| 40 | 4.0 | 19.23 |
| 10 | 2.5 | 22.18 |
| 40 | 2.5 | 20.31 |
| 25 | 1.0 | 6.22 |
| 25 | 4.0 | 17.62 |
| 25 | 2.5 | 14.19 |
| 25 | 2.5 | 11.55 |
| 25 | 2.5 | 14.24 |

**Tableau n°2**

**Figure 1**

**Figure 2**

**Figure 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SHARMA et al.** *Journal of Microbial & Biochemical Technology,* 2014, vol. 6 (4), 202-206
- **ERIKSON N.T.** production of phycocyanin, a pigment with applications in biology, biotechnology, foods and medcine. *Appl Microbiol Biotechnol,* 2008, vol. 80, 1-14
- **HEMLATA T.F.** Screening of cyanobacteria for phycobiliproteins and effect of different environmental Stress on its yield. *Bull Environ Contam Toxicol,* 2009, vol. 83, 509-515
- **LIU Y ; XU L ; CHENG N ; LIN L ; ZHENG CH.** Inhibitory effect of phycocyanin from Arthrospira platensis on the growth of human leukemia K562 cells. *journal of applied phycology,* 2000, vol. 12, 125-130
- **CHEN F ; ZHANG Y ; GUOS.** Growth and phycocyanin formation of Spirulina platensis in photoheterotrophic culture. *Biotechnology Letters,* 1996, vol. 18 (5), 603-608
- **REMZIYE AYSUN KEPEKÇI ; SAADET DEMIRÖRS SAYGIDEGER.** Enhancement of phenolic compound production in Spirulina platensis by two-step batch mode cultivation. *J Appl Phycol,* 2012, vol. 24, 897-905
- **GOUPY J.** Introduction aux plans d'expériences. 2001